# EUROPEAN PATENT APPLICATION

(11) **EP 3 023 115 A1**
(43) Date of publication of application: **25.05.2016**
(21) Application number: 15194892.4
(22) Date of filing: 17.11.2015
(51) Int. Cl.: A61M 5/32, A61B 5/15, A61B 5/153

(54) **SAFETY SYRINGE ASSEMBLY AND REPLACEABLE BASE UNIT THEREOF**

(30) Priority: 20.11.2014 TW 103140171
(71) Applicant: Chou, Yu-Tang, Taoyuan City (TW)
(72) Inventor: Chou, Yu-Tang, Taoyuan City (TW)
(74) Representative: Viering, Jentschura & Partner mbB Patent- und Rechtsanwälte

(57) **Abstract**

A safety syringe assembly and a replaceable base unit are disclosed. The replaceable base unit of the present invention includes a base portion, a needle cover and a disengagement actuator. The base portion is configured to detachably connect with a syringe holder and has a through-hole structure, which is used to connect with an external needle. The needle cover's proximal end is connected with the base portion to allow the needle cover to move with respect to the base portion, enabling it to contain and cover the syringe needle. The disengagement actuator is configured on the base portion and disengages the base portion from the syringe holder in pressing mode.

## Description

### BACKGROUND OF THE INVENTION

This application claims priority benefit of TW Patent Application Ser. No. 103140171 (filed 2014/11/20), which is hereby incorporated herein by reference its entirety.

### 1. Field of the invention

The invention generally relates to a safety syringe assembly and a replaceable base unit thereof. More particularly, the invention relates to a replaceable base unit that may be quickly disengaged from a syringe.

### 2. Description of the prior art

After a medical person finishes using prior art syringe needle for injection or blood drawing, he must place the cap back on and then discard it so as to prevent the possibility that the used needle may stick into any person's body. However, when placing the cap back on, he is prone to the possibility that the used needle may stick into his body. Therefore, the risk at work for medical profession is heightened.

Therefore, safety needle was invented to address the aforesaid problem. For example, in the invention of Taiwan patent no. M485736, a protective cap was used to allow a medical person to replace the cap back on before the needle is discarded. However, in all of prior art applications, a syringe has to be thrown out along with each needle, generating a huge waste of medical resources.

Whence, there is a need to come up with an invention that allows the medical persons to quickly contain and cover the needle and disengage the needle from the syringe.

### Summary of the invention

To solve the previous technical problems, one objective of the present application is providing a safety syringe assembly with a replaceable base unit.

To achieve the aforementioned objective, the present application provides a replaceable base unit, which is applied in an external syringe holder. The replaceable base unit of the present invention comprises a base portion, a needle cover and a disengagement actuator. The base portion is detachably connected with the syringe holder and has a through-hole structure, which is used to connect with a connective portion of a syringe needle and the inner space of the syringe holder. The needle cover's proximal end is connected with the base portion to allow the needle cover to move with respect to the base portion, enabling it to contain and cover the syringe needle (covering mode). The disengagement actuator is configured on the base portion and disengages the base portion from the syringe holder in pressing mode.

To achieve the aforementioned objective, the present application also provides a safety syringe assembly, which comprises a replaceable base unit and a syringe holder. The replaceable base unit comprises a base portion, a needle cover and a disengagement actuator. The base portion is configured to detachably connect with the syringe holder and has a through-hole structure, which is used to connect with the connective portion of an external syringe needle and the inner space of the syringe holder. The needle cover's proximal end is connected with the base portion to allow the needle cover to move with respect to the base portion, enabling it to contain and cover the syringe needle. The disengagement actuator is configured on the base portion and disengages the base portion from the syringe in pressing mode.

Therefore, after a medical person finishes using the safety syringe assembly, he may use his single hand to move the needle cover into a vertical position and press the disengagement actuator to quickly disengage the base portion from the syringe.

### Brief description of the drawings

For a better understanding of the aforementioned embodiments of the invention as well as additional embodiments thereof, reference should be made to the Description of Embodiments below, in conjunction with the following drawings in which like reference numerals refer to corresponding parts throughout the figures.
Fig. 1 is a perspective view of the replaceable base unit applied in a safety syringe holder of the present invention.
Fig. 2 is an exploded view of the safety syringe assembly of the present invention.
Figs. 3 to 5 are three perspective views illustrating how the safety syringe assembly of the present invention is used.

### Detailed description of the preferred embodiment

The following description is about embodiments of the present invention; however, it is not intended to limit the scope of the present invention.

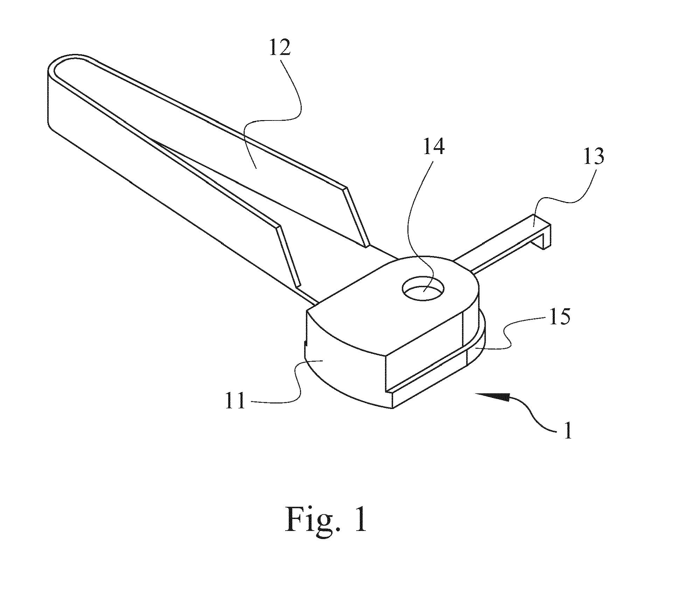

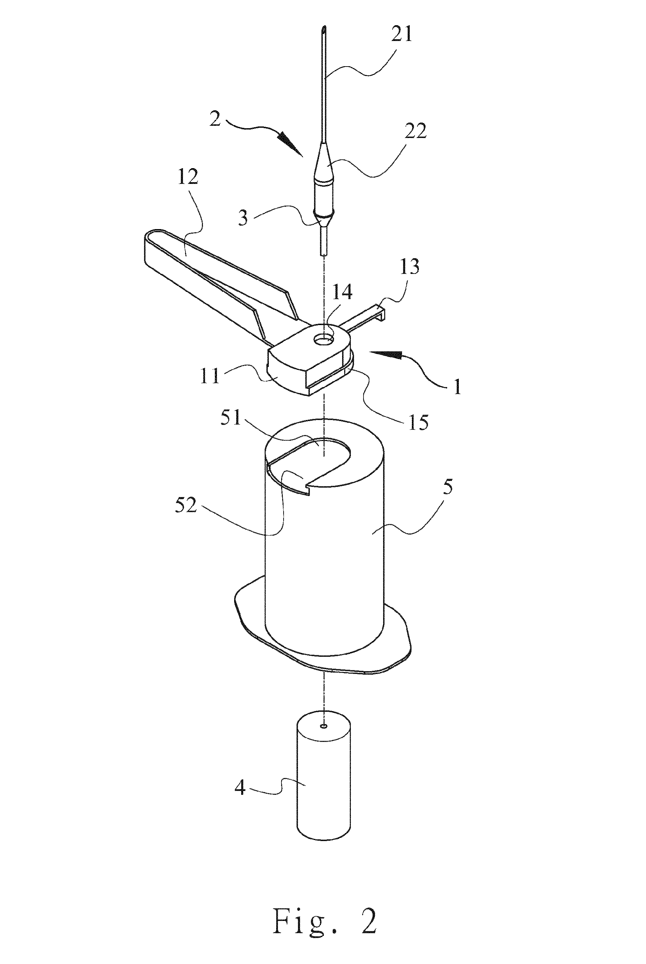

As illustrated in Fig. 1, the replaceable base unit 1 of the present invention which is applied in a syringe holder 5 (Fig. 2) and comprises a base portion 11, a needle cover 12 and a disengagement actuator 13. The base portion 11 may detachably connect with a syringe holder 5. The base portion 11 has a through-hole structure 14 which is use to connect with the connective portion 22 of a syringe needle 2. In use, the proximal end of the needle cover 12 is connected with the base portion 11 and may be pivotally moved into a position to contain and cover the syringe needle 2. The disengagement actuator 13 is configured on the base portion 11 and disengages the base portion 11 from the syringe holder in pressing mode.

The base portion 11 and needle cover 12 may be integrally or removable assembly formed structure. If they are integrally formed, an indented folding line is provides at the junction of them to allow the needle cover 12 to move pivotally with respect to the base portion 11 around the folding line.

A connective portion 15 is configured on the base portion 11 to allow the base portion 11 to detachably connect with the syringe holder 5. For example, the body portion 11 can be detachably embedded at the bottom of the syringe holder 5. The disengagement actuator 13 is connected with the connective portion 15 to allow the base portion 11 to disengage from the syringe holder in pressing mode. The syringe needle 2 may be connected with the through-hole structure 14 in a regular connective manner, a threaded manner or a snap-on manner; or, the through-hole structure 14 may have a tapered design.

The setting positions of disengagement actuator 13 and the needle cover 12 at the base portion 11 are corresponding to positions of user's operating finger of single hand. In other embodiment, the setting positions of the disengagement actuator 13 and the needle cover 12 are corresponding to positions of user's thumb finger and index finger.

Please now refer to Fig. 2, which is an exploded view of the safety syringe assembly of the present invention. The safety syringe assembly comprises the said replaceable base unit 1, the said syringe needle 2, a two-directional connector 3, a collector 4 and the said syringe holder 5. The base portion 11 is detachably embedded at the connective portion 51 of the syringe holder 5 in a more flexible manner. The connective portion 22 of the syringe needle 2 is connected with the two-directional connector 3 and the through-hole structure 14 of the base portion 11. One end of the two-directional connector 3 is housed inside the inner space 52 of the syringe holder 5.

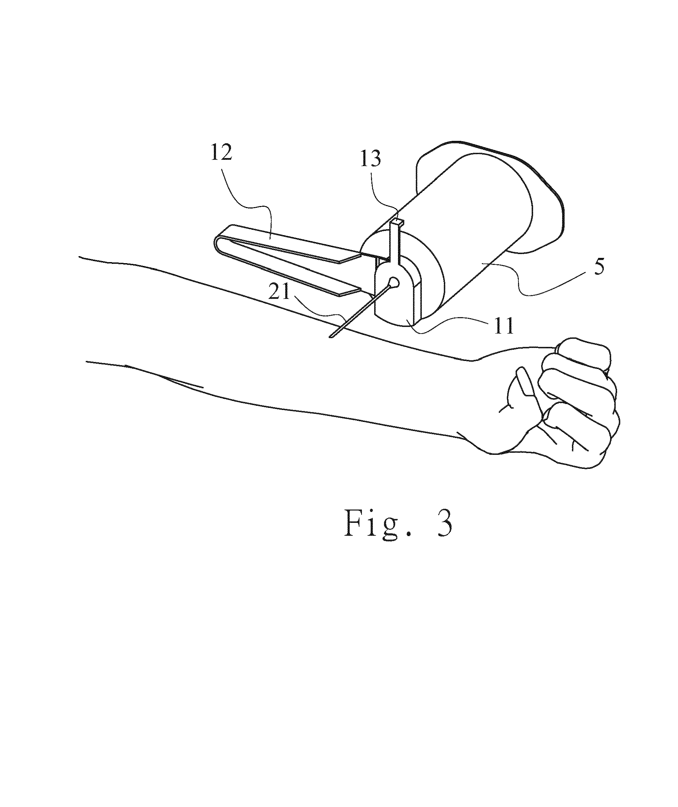

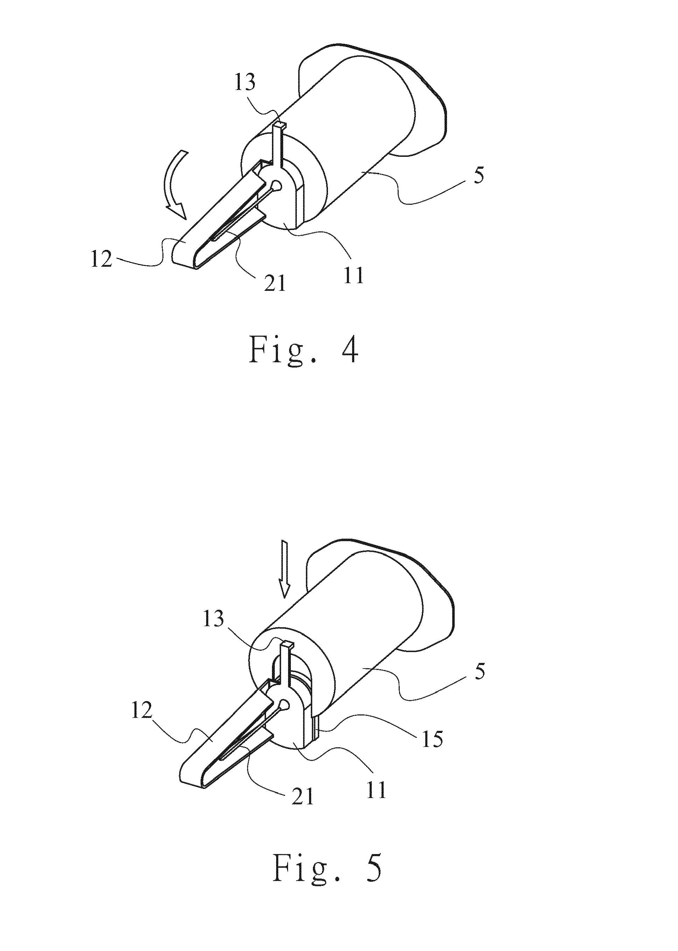

Now, please see Figs. 3 to 5, which illustrate how the safety syringe assembly of the present invention is used. When the safety syringe assembly is used to draw blood, the needle cover 12 is moved to a horizontal position (opening mode) to expose the syringe needle 21. After the needle 21 is inserted into a patient's body, the vacuum collector 4 should be pushed to reach the two-directional connector 3 and then the vacuum collector 4 should be pulled backwards to allow blood to flow into the vacuum collector 4. During the process, a user (such as medical person) may monitor the amount of blood drawn via the transparent part of the base portion 11. After blood is drawn, he may pull the needle 21 out of the patient's body and then pivotally move the needle cover 12 into a vertical position (covering mode) to contain and cover the syringe needle 21. Next, the medical person may press the disengagement actuator 13 to disengage the needle 21 and the base portion 11 from the syringe holder 5 in pressing mode.

In use, first, a medical person may use his thumb or index finger to pivotally move the needle cover 12 to a horizontal position (opening mode). After blood is drawn, he may use his index finger to pivotally move the needle cover 12 into a vertical position (pressing mode) and use his thumb to press the disengagement actuator 13 to disengage the needle 21 from the syringe holder 5. In comparison to the prior art syringe needle design, which requires two hands to operate, the safety syringe assembly of the present invention takes only one hand to operate and may allow the syringe to be reused for several times, thus effectively reducing the amount of medical waste.

The above disclosure is related to the detailed technical contents and inventive features thereof. People skilled in this field may proceed with a variety of modifications and replacements based on the disclosures and suggestions of the invention as described without departing from the characteristics thereof. Nevertheless, although such modifications and replacements are not fully disclosed in the above descriptions, they have substantially been covered in the following claims as appended.

## Claims

1. A replaceable base unit, applied in a safety syringe holder, comprising:
a base portion, configured to detachably connect with the external syringe holder, wherein the base portion has a through-hole structure, which is configured to connect with a connective portion of an external syringe needle and a inner space of the syringe holder;
a needle cover, wherein a proximal end of the needle cover is connected with the base portion and pivotally moved into a position to contain and cover the syringe needle; and
a disengagement actuator, which is configured on the base portion and disengages the base portion from the syringe holder in pressing mode.

2. The replaceable base unit as in claim 1, wherein a connective portion is configured on the base portion to allow the base portion to detachably connect with the syringe holder, wherein the disengagement actuator is connected with the connective portion to allow the base portion to disengage from the syringe holder in the pressing mode.

3. The replaceable base unit as in claim 1, wherein the base portion and the needle cover are selected from an integrally formed structure or a removable assembly structure.

4. The replaceable base unit as in claim 1, wherein the base portion further comprises a transparent part to allow a user to monitor the amount of blood drawn during the medical procedure.

5. The replaceable base unit as in claim 1, wherein setting positions of the disengagement actuator and the needle cover at the base portion are corresponding to positions of operating fingers of single hand.

6. The replaceable base unit as in claim 1, wherein setting positions of the disengagement actuator and the needle cover at the base portion are corresponding to positions of user's thumb finger and index finger.

7. A safety syringe assembly, comprising:
a syringe holder; and
a replaceable base unit, which comprises:
a base portion, configured to detachably connect with the syringe holder, wherein the base portion has a through-hole structure, which is configured to connect with a connective portion of an external syringe needle and an inner space of the syringe holder;
a needle cover, whose proximal end is connected with the base portion to allow itself to be pivotally moved into a position to contain and cover the syringe needle; and
a disengagement actuator, which is configured on the base portion and disengages the base portion from the syringe holder in pressing mode.

8. The safety syringe assembly as in claim 7, wherein the base portion is embedded at a connective opening of the syringe holder.

9. The safety syringe assembly as in claim 7, further comprising a two-directional connector, whose one end is connected with the syringe needle's connective portion.

10. The safety syringe assembly as in claim 9, further comprising a collector, wherein an inner space of the collector is conductively connected with the other end of the two-directional connector.
